# EUROPEAN PATENT APPLICATION

(11) **EP 2 143 452 A1**
(43) Date of publication of application: **13.01.2010**
(21) Application number: 09162552.5
(22) Date of filing: 12.06.2009
(51) Int. Cl.: A61M 1/00, A61M 1/04

(54) **Device for thoracic drainage with suction means**

(30) Priority: 11.07.2008 IT MI20081268
(71) Applicant: Gibertoni, Lucio, 41037 Mirandola (Modena) (IT); Gibertoni, Andrea, 41037 Mirandola (Modena) (IT)
(72) Inventor: Gibertoni, Lucio, 41037 Mirandola (Modena) (IT); Gibertoni, Andrea, 41037 Mirandola (Modena) (IT)
(74) Representative: Modiano, Micaela Nadia

(57) **Abstract**

A device for thoracic drainage with suction means, comprising a collection container (2) that defines at least one chamber that can be connected to a drainage catheter (13) and to portable suction means (20) with a suction source connected to autonomous power supply means, the device further comprising a display (30) for indicating air losses calculated by correlating the volume of the air evacuated from the patient with the period of operation of the suction means and with the value of the suction.

## Description

The present invention relates to a device for thoracic drainage with suction means.

As is known, prior Italian patent 1,348,521, assumed included herein by reference, discloses a device for thoracic drainage with suction means that comprises a collection container in which a chamber is defined that can be connected to suction means and to a drainage catheter.

In the known solution, which has proved to be valid in many respects, the suction means are provided by means of the portable vacuum unit, which has a suction source connected to autonomous power supply means.

With this solution, therefore, it is possible to use the device outside specialized wards, without requiring a connection to centralized vacuum sources.

Moreover, with the device noted above, the patient can move and/or walk without having to renounce maintaining suction.

The known solution suffers an inherent difficulty in the quantitative determination of air losses in the postoperative course of the cardiothoracic patient, losses which are currently assessed by the physician, in practice by observing the intensity of the bubbling of the air that exits from the chest of the patient through a water valve.

This assessment, besides being obviously subjective, makes the diagnosis of air losses very difficult, since the shape of the water valve can vary considerably, making reading less easy.

At the diagnostic level, the lack of a quantitative assessment of air losses makes communication among physicians difficult, with the consequent impossibility to transfer the actual severity of a patient without the interlocutor seeing him in person, and teaching in an academic environment to junior specialist physicians is also difficult, since they must gain their own experience before being able to give correct assessments.

Moreover, the extreme variability of the flow of the air losses of the patient makes an instantaneous measurement of such flow by means of flow meters of the standard type scarcely reliable.

Another aspect to be considered consists in that visual examination of the bubbling of the air that originates from the chest of the patient substantially provides instantaneous information on the flow of air loss of the patient, whereas in order to achieve a diagnosis the physician must log or memorize the intensity of the bubbling at each visit of the patient, assessing the patient's postoperative course only on the basis of qualitative parameters.

The aim of the invention is to solve the problem described above by providing a device which, while maintaining the same features of the device according to the prior cited patent, is particularly compact, as regards dimensions, is versatile and has high-level characteristics of autonomy and portability.

Within this aim, an object of the invention is to provide a device in which it is possible to increase its functionality by providing an objective assessment of the air losses of a patient.

Another object of the present invention is to provide a thoracic drainage device which, thanks to its particular constructive characteristics, is capable of giving the greatest assurances of reliability and safety in use.

Still another object of the present invention is to provide a device that can be obtained easily starting from commonly commercially available elements and materials and is also competitive from a merely economical standpoint.

This aim, and these and other objects that will become better apparent hereinafter are achieved by a device for thoracic drainage with suction means, according to the invention, comprising a collection container that defines at least one chamber that can be connected to a drainage catheter and to portable suction means with a suction source connected to autonomous power supply means, **characterized in that** it comprises a display for indicating air losses calculated by correlating the volume of the air evacuated from the patient with the period of operation of said suction means and with the value of the suction.

Further characteristics and advantages of the present invention will become better apparent from the description of a preferred but not exclusive embodiment of a device for thoracic drainage with suction means, illustrated by way of non-limiting example in the accompanying drawings, wherein:

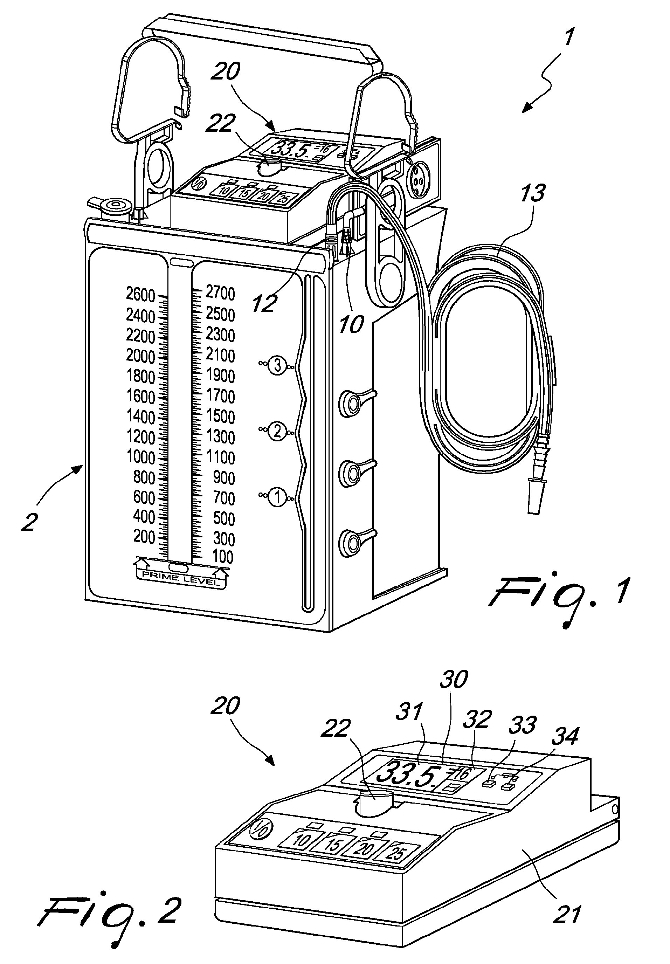

Figure 1 is a schematic perspective view of the thoracic drainage device;
Figure 2 is a perspective view of the portable suction means connected to the collection container.

With reference to the figures, the device for thoracic drainage with suction means, according to the invention, generally designated by the reference numeral 1, comprises a collection container 2, which can be of the single-chamber or double-chamber type or of any other type.

On the collection container there is a port 10 for connection to suction means, described in greater detail hereinafter, and there is a drainage port 12, to which a drainage catheter 13 can be connected.

Advantageously, on the suction line an antibacterial filter is provided which is designed to protect the suction means, which can be reused without risks of contamination.

The suction means comprise a vacuum unit, generally designated by the reference numeral 20, which has a box-like enclosure 21, made of insulating plastics and containing a pump, with a through seat in which a coupling pin 22 that exits from the collection container is inserted.

A suction source, not shown in the drawing, is arranged within the enclosure 21 and is connected to autonomous power supply means, as indicated in the prior cited patent.

Likewise, means for selecting the value of the suction, of the type disclosed in the prior cited patent, are provided.

The characterizing element consists in that a display 30 for displaying air loss data is provided.

The display 30 indicates the air losses of the patient, which are correlated with the period of operation of the suction means and with the value of the suction.

In greater detail, the air that arrives from the patient is conveyed so as to pass through the suction means, which, once the set suction value is known, evacuate a volume of air that is proportional to the activation time of the suction means.

Considering, by way of example, an operating time of one hour, the longer the time for which the pump, that constitutes the vacuum unit, has remained active, the greater the amount of the air that is evacuated.

Substantially, therefore, the device, by using high-precision pumps, is capable of determining how long the pump has operated in relation to the overall power-on time.

This value is converted, by way of conversion factors that depend on the suction value at which the device has worked, into a volume of air that is preferably expressed in liters per hour.

This volume is displayed in a first region 31 of the display and provides an indication of the air evacuated from the chest of the patient, since the drainage system is isolated from the outside environment and therefore there are no other flows involved in the calculation.

The volumetric determination allows to add and average all the variations that can occur during the period being considered without thereby requiring complicated flowmeters and/or processing algorithms.

The value of the air losses related to a preceding period of time, indicated advantageously in the second region 32 of the display, is provided on the first region 31 of the display.

The value is calculated on the basis of one hour, thus achieving an immediate display of the flow in liters per hour.

Of course, conversions to other units of measurement are possible and the display will show the value of the measured flow in the first region 31 and the corresponding period of time in which such flow has been calculated in the second region 32 of the display 30.

A first function key 33 is also provided on the control panel, which allows to scroll back the values calculated in the preceding time intervals, allowing the physician to have a clear and objective assessment of the postoperative course of the patient.

With this method of determining the flow of air losses, there are readings that are spaced one another by a period that is equal to a sensing interval, which is preferably one hour, but there is a second function key 34, which provides the acquired flow value related to a very short preceding period of time, preferably equal to one minute, which is indicated in the first region 31, together with the indication of the measurement time interval, preferably of one minute, indicated in the second region 32.

In practice, a more "instantaneous" reading of the air losses of the patient is provided with respect to the measurement displayed in a relatively long period of time, which provides an average value in a longer time, thus eliminating the variations caused by patient respiration.

The device as described above allows to solve the described problems by providing a quantitative data item regarding the value of the air losses, i.e., a number with which it is possible to associate immediately and uniquely a clinical state of the patient, which can be more or less severe, without depending on approximate assessments whose extent can be judged differently by different physicians.

The device can operate in association with different drainage systems, with one or more chambers, but detection of the flow of the air losses does not depend on the structure of these systems, and therefore the physician can obtain an easily readable reference value, eliminating the visualization difficulties caused by the difference of the drainage systems.

The possibility to read a numeric value, linked to the air losses of the patient, allows easy and straightforward communication between physicians without requiring both to personally visit the patient, and therefore consultation and accordingly a better diagnosis become easier.

Also on a teaching level, teaching to junior specialist physicians can be based on numerical data that indicate unambiguously the severity of the clinical state of the patient, greatly reducing learning times and eliminating personal interpretations of data.

Determining the air losses by means of the principle of conversion between the operating time and the volume of air removed by a high-precision suction pump allows to measure easily the flow of air in a given time interval without the influence of all the involved variables of the postoperative course of a cardiothoracic patient, such as respiratory activity, coughing, systoles, changes of posture and so forth.

An important aspect of the invention consists in that this result is achieved simply, repetitively and reliably without resorting to sophisticated flowmeters or complicated calculation algorithms.

Another important aspect further consists in that a rapid and intuitive visualization of the data related to the preceding hours is obtained, providing the physician with the possibility to assess objectively the postoperative course of the patient.

It should also be noted that the introduction of the device in the same container as the drainage device according to the prior cited patent allows to have a reduced space occupation, with consequent easy transport of the system proper and with a considerable facilitation in transport and walking of the patient.

The invention thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements.

The disclosures in Italian Patent Application no. MI2008A001268, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for thoracic drainage with suction means, comprising a collection container (2) that defines at least one chamber that can be connected to a drainage catheter (13) and to portable suction means (20) with a suction source connected to autonomous power supply means, **characterized in that** it comprises a display (30) for indicating air losses calculated by correlating the volume of the air evacuated from the patient to the period of operation of said suction means (20) and to the value of the suction.

2. The drainage device according to claim 1, **characterized in that** said display (30) defines a first region (31) for displaying the value of the air losses in a period being considered and a second region (32) for indicating the corresponding period of time.

3. The drainage device according to the preceding claims, **characterized in that** it comprises function keys (33, 34) in order to vary the values indicated in said first region (31) and said second region (32).

4. The drainage device according to one or more of the preceding claims, **characterized in that** it comprises a first function key (33) for scrolling backward through the values calculated in the preceding time intervals.

5. The drainage device according to one or more of the preceding claims, **characterized in that** it comprises a second function key (34) for indicating the value of the flow acquired in a period of time that is shorter than said period being considered.
